# EUROPEAN PATENT APPLICATION

(11) **EP 2 260 783 A1**
(43) Date of publication of application: **15.12.2010**
(21) Application number: 10275060.1
(22) Date of filing: 09.06.2010
(51) Int. Cl.: A61B 18/20

(54) **Optical radiation treatment**

(30) Priority: 10.06.2009 GB 0909953
(71) Applicant: The Dezac Group Limited, Cheltenham Gloucestershire GL50 1SS (GB)
(72) Inventor: Herbert, Kevin, Cheltenham, Gloucestershire GL51 0PQ (GB)
(74) Representative: Newell, William Joseph

(57) **Abstract**

A light treatment apparatus includes a source of radiation controlled by a pulse modulator to generate a train of discrete spaced optical pulses which generate in the skin or heat pulse envelope of flattened from whereby the temperature of a selected one or more components at the treatment site remains below a preset temperature without requiring cooling of the skin.

## Description

This invention relates to methods and apparatus for treatment of the human or animal body by exposing a treatment site to optical radiation. The term "Optical Radiation" is used to include any radiation that obeys optical properties and includes radiation from the infrared waveband and the ultraviolet wavebands. The term includes coherent and incoherent radiation as well as broad spectrum and narrow spectrum radiation.

The use of optical radiation whether laser or intense pulsed light ("IPL") is known for a variety of cosmetic and therapeutic treatments including hair removal, treatment of vascular and pigmented birthmarks, treatment of broken capillaries to name but a few. In a known form of IPL device for hair removal, a capacitor is charged up to store electrical energy, which is then discharged through a flash lamp (typically a xenon flash lamp). This produces a rounded pulse of optical energy which is absorbed in the skin, heating the skin and heating the hair bulb or "root" which usually contains the highest concentration of melanin. The light energy is converted to heat energy thus vaporising or at least killing the root and the remainder of the hair follicle. It has been determined that the rounded pulse meant that there was a substantial variation in the magnitude of the current flowing through the flash lamp during the discharge which in turn meant that the wavelength shifted during the duration of the pulse. Attempts have been made to overcome this problem by providing a switch between the capacitor and the flash lamp and controlling the switch to provide a relatively constant current output utilising a flywheel diode. W02009/013524 discloses a device in which an optical sensor is provided to detect the optical output from the flash lamp and to control the energy supplied to the flash lamp to maintain a given optical output. Although such devices aim to provide a generally uniform level of current to the flash lamp and a generally uniform optical output respectively, both devices essentially deliver a single pulse or dose of radiation which is deposited into the skin and which creates a heating effect. Our studies have shown that such single dose or monopulse arrangements, both deliver energy into the skin in a manner which causes a rounded heat pulse envelope, the peak of which tends to exceed the temperature required for the given skin treatment. In other words, with prior art devices, there is an overshoot in the heat pulse which has to be remedied either by active cooling of the applicator head or by the use of a cooling gel, to prevent damage to the skin. Although the above discussion has centred on the use of IPL devices for hair removal, similar considerations apply in those devices for treatment of different conditions. The requirement is to provide an effective therapeutic dose of optical radiation into the skin which is sufficient to apply the therapeutic effect, but without causing damage to the skin by overheating.

Accordingly, in one aspect, this invention provides apparatus for treatment of the human or animal body by exposing a treatment site to optical radiation to cause heating of said site and/or elements therein, said apparatus comprising:
a source of pulsed optical radiation;
a pulse modulator for controlling the pulse width and the pulse spacing of the output of said source;
wherein the pulse modulator is adapted in use to deliver a pulse treatment train comprising a series of discrete spaced pulses such that, over the duration of the train, the heat pulse envelope experienced at the treatment site is of generally flattened extended form, whereby the temperature at the treatment site or of one or more selected elements thereof remains below a preset temperature, without requiring cooling of the skin.

In another aspect, this invention provides apparatus for treatment of the human or animal body by delivering to a treatment site an effective dose of optical radiation sufficient to cause heating of said site and/or elements therein to provide said treatment, said apparatus comprising:
a source of pulsed optical radiation;
a dose control for controlling said source of pulsed optical radiation,
whereby said dose is delivered as a series of discrete spaced pulses together making up said effective dose.

In a further aspect, this invention provides apparatus for treatment of the human or animal body by exposing a treatment site to optical radiation to cause heating of said site and/or elements therein, said apparatus comprising:
a source of pulsed optical radiation;
a pulse modulator for controlling the pulse width and pulse spacing of the output of said source;
wherein the pulse modulator delivers a pulse train made up of a plurality of discrete spaced pulses each of duration less than 5ms and spaced by no more than 100ms. The pulse train is preferably of duration greater than 150ms.
The limit on the pulse spacing may depend on the given fluence of the radiation. The pulse spacing in a preferred arrangement is less than 50ms.

In the above aspects it will be noted that, contrasted with the prior art arrangements, the treatment radiation is delivered as a series of pulses as opposed to the monopulse arrangements between the pulses the optical output is zero or nearly 80. Delivery of the optical energy in a series of pulses enables the heat pulse envelope to be optimised so that the treatment site, or selected elements thereof, do not exceed the desired temperature(s) yet still allow an effective amount of energy to be delivered to effect the treatment.

There are number of ways in which the appropriate parameters for the duration of the pulse train or dose, the width and spacing of the pulses making up the dose can be determined. These may be determined empirically for a range of different skin and hair types, type of hair, gender, treatment type etc and the appropriate values provided on a chart or lookup table. A user can then determine the appropriate parameters to set depending on the data in the chart/lookup table.

In the preferred apparatus described herein, it has been found that significantly lower fluences, typically less than 5.0J/cm2 and preferably around 2.7J/cm2, can be used. Traditional hair removal devices use a fluence greater than 10J/cm2 and usually in the region of 30J/cm2. It is believed that the reason for this is because the energy is delivered over a much longer pulse (train) width (200ms compared to 20ms).

In another arrangement, a suitable detector may be provided which detects the temperature of the treatment site and/or of one or more selected elements therein, and the output of such a detector may be used to control the parameters. The detector may also act to prevent operation of the device outside of acceptable predetermined parameters.

Accordingly, in yet another aspect, this invention provides apparatus for treatment of the human or animal body by exposing a treatment site to optical radiation to cause heating of said site and/or elements therein, said apparatus comprising:
a source of optical radiation;
a detector for detecting the temperature of the treatment site and/or one or more selected elements therein;
a controller for controlling the source of optical radiation,
wherein the controller is responsive to said detector to control the source of optical radiation to maintain the temperature of the treatment site and/or one or more selected elements therein below a predetermined threshold temperature.

Various options for preferred features that may be incorporated in one or more of the above aspects will now be described.

Where intended for use in a hair treatment device, the detector may determine the temperature of the skin, or hair follicles in the treatment site, or both.

The detector, or another detector viewing the treatment site, may be used to determine or device parameters of the treatment site such as skin type or tone; hair colour or hair density etc.

Furthermore, the detector for detecting the temperature of the treatment site may also be used to determine whether or not the device is in close proximity to an area of skin. Thus a safety circuit may inhibit operation of the device unless the detector output is consistent with the detector having being applied to the skin, or brought to within an allowable treatment range.

The maximum temperature for the skin is typically about 42°C and the temperature required at the hair root to effect hair removal is of the order of 80°C.

Typical parameters for a dose for hair removal would be a pulse train of length over 200ms and preferably around 300ms. Each pulse in the train is preferably of duration less than 5ms and ideally around 1ms. The spacing between the pulses is variable to enable the appropriate heat pulse envelope to be produced; the spacing may be up to 50ms but more preferably will typically be in the region of between 5 and 10ms. Additionally, the pulse ON time can be varied to adjust the amount of energy deposited. Thus it may be varied up to e.g. a maximum of 5ms.

It has been found that a wavelength of around 800nm is desirable for this application. Also it has been found that the lower the voltage applied to discharge lamp, the more radiation at around 800nm is produced by the discharge lamp. However, if the voltage applied is too low then the lamp will not flash. We have also found that it is possible to run a discharge lamp at a significantly lower voltage than is conventional for discharge lamps for this purpose, thus saving in cost, energy consumption, and heat build up in any optical filter that is used. By reducing the voltage to significantly below 300V, more preferably below 200V and ideally around 150V, we have found that more of the desired 800nm wavelength output is produced. Also of course the lower fluences required facilitate this reduction in voltage. Generally, such a low voltage does not produce the desired treatment effect (e.g. epilation) but the discrete spaced pulses rectify or improve the effect when the lower voltages are employed.

The apparatus preferably includes a number of safety devices to ensure safe operation of the apparatus. Thus, as mentioned above, where the device includes a heat detector, the output of the heat detector may be used to determine whether the device is in contact with or in close proximity to the skin. In another arrangement, the apparatus may have an applicator head with an aperture through which the optical radiation passes and a plurality of skin contact electrodes may be provided around the aperture with the safety circuit inhibiting operation of the apparatus unless all the skin contact electrodes are in contact with the skin. For example, the skin contact electrodes may be arranged as four quadrant electrodes. In a preferred arrangement the contact electrodes may be slightly recessed. This makes it a requirement that the device is in contact with the skin and additionally under slight pressure before the skin deforms into the recess to touch the detector. This makes it more difficult to "fool" the skin detectors.

Furthermore, where the device includes a heat detector which produces a pixellated image or otherwise generates a 2-D image, a dosage calculating circuit may determine one or more parameters such as the hair density or the skin type and use this to calculate an appropriate dosage. The heat detector may monitor the temperature of both the skin and the hair or parts thereof with these measurements being used to control the optical radiation dose appropriately to ensure that a treatment temperature for the hair is exceeded but that the skin temperature does not rise above a safe threshold.

The apparatus may include a further light emitting element arranged to emit a high intensity beam of radiation non-injurious to the eye in generally the same direction as said source of optical radiation, thereby to deter a user from aiming the apparatus towards their eye, and/or to initiate a blink reflex.

The high intensity beam is designed to be non-injurious in the sense that whilst it might be unpleasant to look into the beam, no permanent injury is caused by that beam.

Preferably the high intensity beam is turned on as soon as the apparatus is powered and before the source of optical radiation is actuated. In most instances, the source of optical radiation will be fired on demand using a trigger or the like. The light emitting element preferably emits light at a wavelength to which the eye is particularly sensitive, such as light of wavelength of the order of 500-600nm and ideally around 555nm. The light-emitting element may comprise an LED designed to provide light at the required frequency.

Preferably the apparatus includes a timer interrupting operation of the device after a preset period. This feature prevents overexposure of the skin which could result in burns, and forces the user to remove the apparatus from the targeted area between uses, thereby giving sufficient time for the area to cool, and also making relocation of the device onto the exact same area unlikely. The contact or proximity sensor of the various types described above may be used in conjunction with a timer so that, on expiry of the predetermined period, re-energisation of the source of optical radiation is allowed only after the apparatus has been removed from the skin for a preset period, and reapplied to the skin.

Whilst the invention has been described above, it extends to any inventive combination of the features set out above or in the following description or claims. The invention also extends to the methods of operation as herein disclosed.

The invention may be performed in various ways and, by way of example only, an embodiment thereof will now described in detail, reference being made to the accompanying drawings in which:
Figure 1 is a schematic view of the of light output monopulse and the consequent heat pulse in a first type of prior art IPL device;
Figure 2 is a schematic view of the light output monopulse and the consequent heat pulse in a second type of prior art IPL device;
Figure 3 is a block diagram of a first embodiment of a device in accordance with this invention;
Figure 4 is view similar to Figures 1 and 2 but showing the multiple light output pulses and the corresponding heat pulse from the device of Figure 3 in accordance with the invention;
Figure 5 is a more detailed view similar to Figure 4 showing the relation between the light output pulses and the heat pulse in an embodiment of this invention;
Figure 6 is a simple diagram showing a principle of operation of a closed- loop device in accordance with a more modern of this invention;
Figure 7 is a more detailed view illustrating operation of the controller of the apparatus;
Figure 8 is a graph showing the skin and hair temperature heat pulses for an embodiment of this invention, and
Figure 9 is a schematic view of another embodiment of this invention.

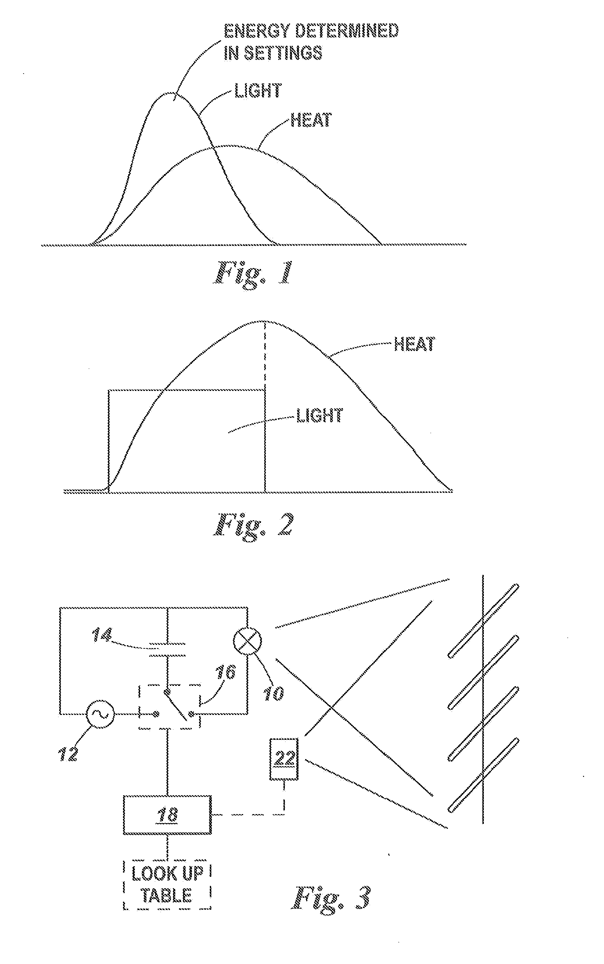

Referring to Figures 3 to 7 of the drawings, the embodiments illustrated therein are intended for use as a light treatment device designed to deliver effective therapeutic doses of optical radiation onto and into the skin but controlled to avoid generation of excess temperatures and thus the need for cooling.

Referring initially to Figure 3, in a first embodiment, a drive circuit for the discharge lamp 10 comprises a power source 12 a capacitor 14 and a high voltage switch 16 arranged alternately to connect the capacitor 14 to the power source for charging and to the lamp 10 for discharging through the lamp 10 to create a light pulse. The high voltage switch 16 is controlled by a controller 18 which controls the three main variables of the circuit namely the width and spacing of the voltage pulses supplied from the capacitor 14 to the lamp 10 as well as the overall length of pulse train in any one treatment or "dose". It should be noted that the pulse width and pulse spacing may each vary in magnitude during any given dose. In the table below we set out typical ranges.

| **Parameter** | **Range** |
|---|---|
| Pulse width | less than 5ms, typically about 1 ms |
| Pulse spacing | up to 50ms, typically 5ms to 10ms |
| Pulse train/dose | greater than 200ms, typically above 250ms and preferably about 300ms |

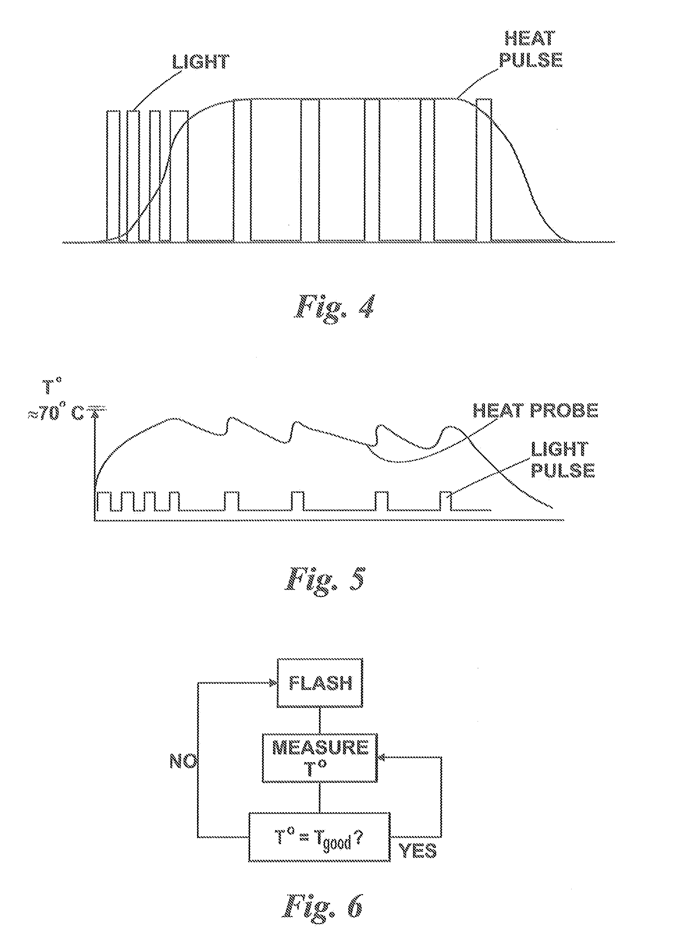

Figure 4 shows schematically the relationship between the light pulses and the heat pulse. Compared to Figures 1 and 2 it will be seen that the light is delivered in a series of relatively narrow, spaced, pulses, here of substantially uniform pulse width, but with the spacing varying during the duration of the treatment dose. Between the pulses the optical output returns to zero or a quiescent level such that the pulses are discrete and do not merge into each other. The pulses are spaced closely together at the beginning of the dose in order to quickly raise the temperature of the heat pulse to the required value (about 80°C) whereupon the spacing between the pulses increases to deliver sufficient heat to the treatment site to maintain the temperature at a substantially uniform level. In fact of course there will be some variation in the temperature as shown in the more detailed graph of Figure 5. In this way it will be noted that the overshoot or peak in Figures 1 and 2 is avoided and they flattened heat pulses achieved.

In practice, the amount of light that needs to be delivered depends on numerous factors including the particular treatment being applied but also the skin pigmentation of the user, the hair density and colour and so on. The energy requirements vary therefore between different skin types etc. The parameters of the pulse train typically therefore need to be set for each different type of use. In a simple version of this device, a lookup table 20 is constructed which indicates the pulse train parameters required for each of a number of different combinations of skin type hair type area of the body etc. For each of these combinations appropriate pulse train parameters are designed to ensure that sufficient heat energy is deposited into the skin to achieve the required effect whilst not exceeding the skin temperature threshold. There are of course a number of ways in which a lookup table can be suitably produced; for example a study may be undertaken of suitable subjects having the various combinations of hair and appropriate pulse train parameter combinations designed to produce the required effect.

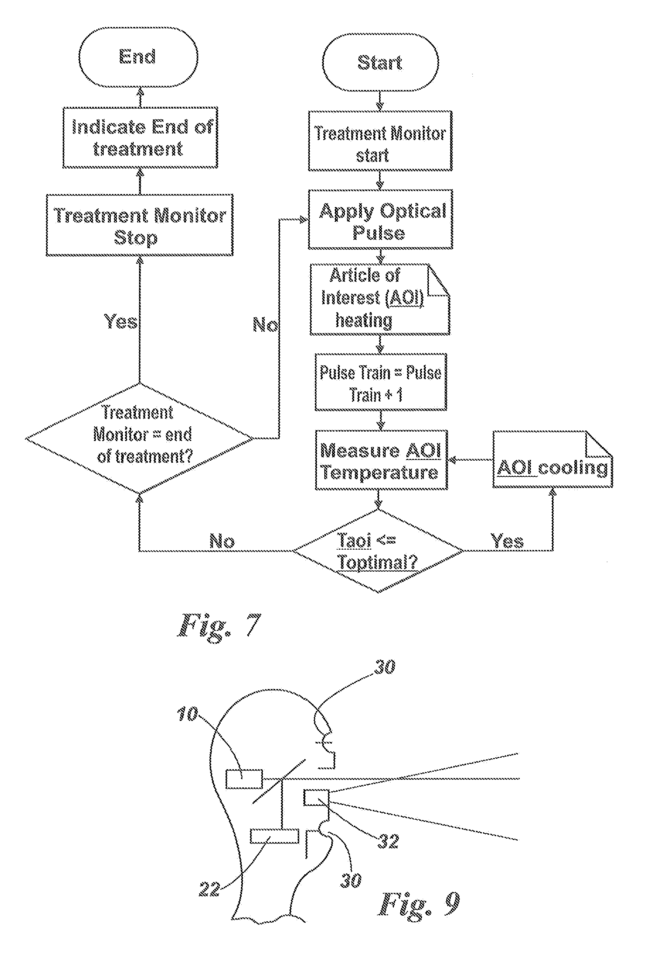

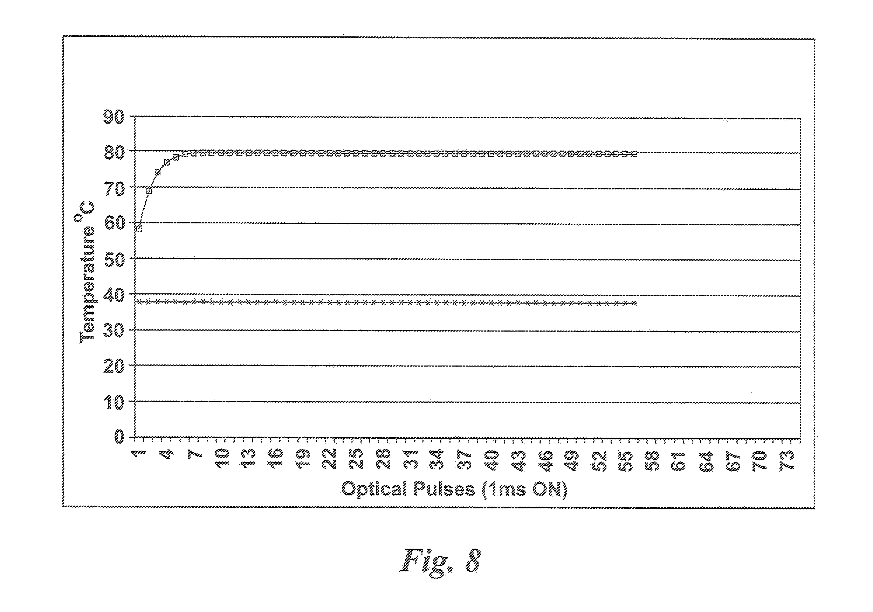

However, in a closed loop device, the apparatus may be provided with a heat detector 22, which monitors the area of skin exposed to the optical radiation from the lamp 10 and measures the temperature of the site. Depending on the nature of the detector this may simply be a spot reading or it may be a two- dimensional thermal image of the treatment site. In the latter case, temperature readings may be taken of the skin or the hair follicles. In either event the output from the detector 22 is provided to the controller, which adjusts the pulse train parameters. For example, as soon as the detector determines that the appropriate treatment temperature has been reached, it may lengthen the pulse spacing. It will be appreciated that the usual control algorithms such as PID control may be used here. Figures 6 and 7 show suitable routines. Figure 8 illustrates the temperature profiles of the skin (lower trace) and the hair (upper trace) for an embodiment of this invention from which it will be seen that both rise sharply but quickly reach respective steady temperatures.

In another arrangement, the controller may look at a suitable combination of the skin temperature and the hair temperature and adjust the pulse train parameters accordingly. In addition, where a two dimensional detector is used, the controller may determine the number and size of the hairs present in a treatment site and adjust the pulse train parameters correspondingly. It will be appreciated that where the density of hair is lower, less energy may be required to perform effective treatment.

In one arrangement, the controller may monitor both the skin temperature and the hair temperature and adjust the pulse train parameters to ensure that the hair temperature reaches 80°C as soon as possible whilst ensuring that the skin temperature remains below about 40°C.

The controller may also run a series of safety measures to ensure that the device is not misused. Thus the controller may inhibit the operation of the device if the signal produced by the detector is not commensurate with the device having being placed firmly against a treatment site. Furthermore, as seen in Figure 9, a set of contact electrodes 30 may be provided which detect contact with the skin around the treatment site and the controller may inhibit operation if skin contact is not detected by all the electrodes. The light emitted by the lamp 10 contains sufficient energy potentially to cause eye damage if directed into the eye. In order to prevent this, a blink aversion lamp 32 may be provided which emits a broad beam of radiation of beam width greater than that of the lamp 10 to trigger a blink reflex to cause the user or others involuntarily to blink if the device is pointed towards them. The controller may also include suitable timers and control circuitry to ensure that, on completion of a dose, a user has to remove the device from the skin and to reapply it before a further dose can be delivered. Where the device includes a 2D heat detector 22 the controller may use it to see if there are any "hot" hairs in the treatment site and to deny treatment if this is the case. The heat signature depends on the hair thickness; in the worst case (thick) the hair temperature falls by half every 50ms. In the skin the heat signature depends on skin colour, in the worst case (tanned) the temperature drops by half in 25ms.

## Claims

1. Apparatus for treatment of the human or animal body by exposing a treatment site to optical radiation to cause heating of said site and/or elements therein, said apparatus comprising:
a source (10) of pulsed optical radiation;
a pulse modulator (16, 18) for controlling the pulse width and the pulse spacing of the output of said source;
wherein the pulse modulator (16, 18) is adapted in use to deliver a pulse treatment train comprising a series of spaced pulses such that, over the duration of the train, the heat pulse envelope experienced at the treatment site is of generally flattened extended form, whereby the temperature at the treatment site, or of one or more selected elements thereof, remains below a preset temperature, without requiring cooling of the skin.

2. Apparatus for treatment of the human or animal body by delivering to a treatment site an effective dose of optical radiation sufficient to cause heating of said site and/or elements therein to provide said treatment, said apparatus comprising:
a source (10) of pulsed optical radiation;
a dose control (18) for controlling said source of pulsed optical radiation,
whereby said dose is delivered as pulse train comprising a series of pulses together making up said effective dose.

3. Apparatus for treatment of the human or animal body by exposing a treatment site to optical radiation to cause heating of said site and/or elements therein, said apparatus comprising:
a source (10) of pulsed optical radiation;
a pulse modulator (16, 18) for controlling the pulse width and pulse spacing of the output of said source;
wherein the pulse modulator (16, 18) delivers a pulse train made up of a plurality of pulses each of duration less than 5ms and spaced by no more than 100ms.

4. Apparatus for treatment of the human or animal body by exposing a treatment site to optical radiation to cause heating of said site and/or elements therein, said apparatus comprising:
a source (10) of optical radiation;
a detector (22) for detecting the temperature of the treatment site and/or
one or more selected elements therein;
a controller (18) for controlling the source of optical radiation,
wherein the controller (18) is responsive to said detector (22) to control the source (10) of optical radiation to maintain the temperature of the treatment site and/or one or more selected elements therein below a predetermined threshold temperature.

5. Apparatus as claimed in claim 4, wherein said detector (22) is operable to detect the temperature of at least one of the skin and the hair follicles.

6. Apparatus as claimed in claim 4 wherein the processor (18) uses the detector (22) to determine whether the device is in contact with or in close proximity to an area of skin.

7. Apparatus as claimed in claim 6, wherein the controller (18) inhibits operation of the device unless the detector output is consistent with the apparatus having been applied to the skin, or being within a predetermined allowable treatment range.

8. Apparatus as claimed in any of Claims 1 to 3, wherein the pulse train is of duration over 200ms.

9. Apparatus as claimed in Claim 8, wherein the pulse train is of about 300ms duration.

10. Apparatus as claimed in any of Claims 1 to 3, wherein each pulse of the train is of duration less than 5ms, and ideally around 1 ms.

11. Apparatus as claimed in any of Claims 1 to 3, or 8 to 10, wherein said pulse spacing is variably controlled by the controller (18).

12. Apparatus as claimed in any of Claims 1 to 3, or 8 to 11, wherein said pulse spacing is less than or equal to 50ms, and more preferably between 5ms and 10ms.

13. Apparatus as claimed in any of the preceding Claims, wherein the apparatus comprises an applicator head with an aperture through which the optical radiation passes, and a plurality of skin contact electrodes provided around the aperture, with the operation of the apparatus being inhibited unless all the skin contact electrodes are in contact with the skin.

14. Apparatus as claimed in Claim 4, where the temperature detector produces a 2-D image, said apparatus further including a dosage calculating circuit for processing the image data from said temperature detector to calculate an appropriate dosage.

15. Apparatus as claimed in any of the preceding Claims, including a further light emitting element arranged to emit a high intensity beam of radiation non-injurious to the eye in generally the same direction as said source of optical radiation, thereby to deter a user from aiming the apparatus towards their eye.
